# EUROPÄISCHE PATENTSCHRIFT

(11) **EP 0 700 249 B1**
(45) Veröffentlichungstag und Bekanntmachung des Hinweises auf die Patenterteilung: **22.07.1998**
(21) Anmeldenummer: 94918330.5
(22) Anmeldetag: 17.05.1994
(51) Int. Cl.: A01N 47/44, A61L 2/00, A61K 31/155

(54) **ANTIINFEKTIVUM**
ANTI-INFECTIVE AGENTS
AGENTS ANTI-INFECTIEUX

(30) Priorität: 26.05.1993 DE 4317477
(43) Veröffentlichungstag der Anmeldung: 13.03.1996
(73) Patentinhaber: Fresenius AG, 61350 Bad Homburg (DE)
(72) Erfinder: KIRSCHNER, Ulrich, D-64546 Mörfelden-Walldorf (DE); JETHON, Frank, D-61350 Bad Homburg v.d.H. (DE); RAUCH, Frank, D-61352 Bad Homburg v.d.H. (DE)
(74) Vertreter: Luderschmidt, Schüler & Partner GbR
(86) Internationale Anmeldenummer: EP9401587
(87) Internationale Veröffentlichungsnummer: WO9427440

(56) Entgegenhaltungen:
- EP-A- 0 180 309
- EP-A- 0 450 117
- WO-A-86/02001
- DE-A- 3 537 627
- GB-A- 702 268
- GB-A- 1 152 243
- THE JOURNAL OF APPLIED BACTERIOLOGY, Bd.54, 1983 Seiten 345 - 353 P.BROXTON ET. AL. 'A study of the antibacterial activity of some polyhexamethylene biguanides towards Escherichia coli ATCC 8739'

## Beschreibung

### Technisches Gebiet

Die vorliegende Erfindung betrifft ein Antiinfektivum auf Basis von Poly(hexamethylen)biguanid, insbesondere in Form eines Wundantiseptikums und/oder eines Wundbehandlungsmittels und/oder einer Lösung zur intravenösen Verabreichung, sowie die Verwendung des Poly(hexamethylen)biguanids zur Herstellung eines Antiinfektivums, wie eines Desinfektionsmittels, eines Wundantiseptikums und/oder Wundbehandlungsmittels sowie eines antimikrobiellen, antiviralen und/oder antiparasitären Mittels, vorzugsweise für intravenöse Verabreichung.

### Stand der Technik

Aus dem Stand der Technik ist bekannt, daß Poly(hexamethylen)biguanid (PHMB) eine bakterizide und fungizide Wirkung aufweist (vgl. z.B. GB-PS 12 02 495). PHMB wird daher in vielen Bereichen als Desinfektionsmittel z.B. in Form von Lösungen oder Sprays eingesetzt. Anwendung finden sie z.B. in der Lebensmittelindustrie zur Reinigung und Desinfektion von Räumen und Geräten, zur Stabilisierung von Getränken und zur Reinigung und Stabilisierung von Wasser, z.B. auch in Schwimmbädern zur Bekämpfung von Algen- und Bakterienwuchs. Aus der DE-OS 35 37 627 ist bekannt, daß durch Kombination von PHMB mit einem Molekulargewicht von 1.700 bis 2.500 mit einer geringen Menge Polyethylenglycol Desinfektionsmittel erhalten werden, die auch als lokale Antiseptika in der Wundbehandlung eingesetzt werden. Als PHMB geeignet ist gemäß dieser DE-OS z.B. das PHMB, das als Hydrochlorid unter dem Warenzeichen Vantocil^{R} IB von der ICI vertrieben wird.

In der EP 04 50 117 wird eine Ringerlösung und deren Anwendung als bakterizid wirkendes lokales Wundbehandlungsmedikament beschrieben, wobei die lactatfreie Ringerlösung zusätzlich 0,1 % - 0,2 % eines Konzentrates gelöst enthält, das aus einer 20 %igen wässrigen Poly(hexamethylen)biguanid-Hydrochlorid-Lösung besteht, in der pro 100 ml 1 g Polyethylenglycol mit einem Molekulargewicht von etwa 4.000 gelöst ist. Als PHMB wird ebenfalls lediglich das unter dem Warenzeichen Vantocil^{R} IB der Firma ICI vertriebene Produkt als geeignet beschrieben. Unter dem Warenzeichen Lavasept^{R} ist ein Produkt für die Wundheilung bekannt, wobei das Lavasept-Konzentrat eine wässrige Lösung von 20 Gew.-% PHMB und 1 Gew.-% Polyethylenglycol 4.000 darstellt und das PHMB das Handelsprodukt Vantocil^{R} IB der Firma ICI ist.

In der US-PS 47 58 595 (1) werden Lösungen beschrieben, die eine mikrobizid oder fungizid wirksame Menge eines Biguanides oder eines wasserlöslichen Salzes desselben in einer Menge von 0,000001 bis 0,0003 Gew.-% enthalten, die für Kontaktlinsen, ophthalmologische Produkte und dermatologische Formulierungen, die nahe des Auges angewendet werden, eingesetzt werden.

Aus der GB-PS 1 432 345 (2) sind Zusammensetzungen für die Verwendung in Verbindung mit Augen und Kontaktlinsen bekannt, die mindestens ein ophthalmisch akzeptables polymeres Biguanid enthalten.

### Darstellung der Erfindung

Die Aufgabe der vorliegenden Erfindung ist es, ein Antiinfektivum bereitzustellen, das insbesondere als Wundantiseptikum und/oder als Wundbehandlungsmittel sowie zur Prophylaxe und Behandlung von Infektionen, wie als antimikrobielles, antivirales und/oder antiparasitäres Mittel eingesetzt werden kann, und das den bekannten Desinfektionsmitteln bzw. antimikrobiellen Mitteln gegenüber hinsichtlich der Wirksamkeit überlegen ist und gleichzeitig eine geringere Toxizität besitzt.

Erfindungsgemäß wurde überraschend gefunden, daß Poly(hexamethylen)biguanid mit einem Molekulargewicht, das höher ist als das Molekulargewicht des Poly(hexamethylen)biguanid, wie es in dem aus der DE-OS 35 37 627 und der EP 04 50 117 bekannten Stand der Technik eingesetzt wurde, bzw. daß Poly(hexamethylen)biguanid, wie es in dem aus der DE-OS 35 37 627 und der EP 0 450 117 bekannten Stand der Technik eingesetzt wurde, aus dem jedoch die niedermolekularen Bestandteile abgetrennt worden waren, eine dem bisher eingesetzten Poly(hexamethylen)biguanid gegenüber gesteigerte mikrobizide Wirkung aufweist, die gleichermaßen an Bakterien sowie an Pilzen und Viren zu beobachten ist. Damit ergibt sich - verglichen mit dem bisher eingesetzten PHMB - für das PHMB mit höherem Molekulargewicht bzw. für das von den niedermolekularen PHMB-Bestandteilen befreite PHMB eine geringere Toxizität bei gleich großer Wirksamkeit. Ferner wurde überraschend gefunden, daß die schweren zentralnervösen Störungen, wie sie bei dem bisher eingesetzten PHMB beobachtet werden, bei dem PHMB mit dem höheren Molekulargewicht nicht auftreten.

Erfindungsgemäß wurde überraschend gefunden, daß diese - von Nebenwirkungen auf das Zentralnerversystem freie - gesteigerte Wirksamkeit und geringere Toxizität mit Poly(hexamethylen)biguanid mit einem mittleren Molekulargewicht M_{w} im Bereich von 2.900 bis 15.000 erzielt werden kann. Besonders bevorzugt ist ein Poly(hexamethylen)biguanid mit einem mittleren Molekulargewicht M_{w} im Bereich von 3.000 bis 8.000 und insbesondere mit einem PHMB mit einem mittleren Molekulargewicht M_{w} im Bereich von 3.200 bis 5.000, z.B. PHMB mit einem mittleren Molekulargewicht M_{w} von 3.500, von 4.000 oder von 4.500. Die Molekulargewichtsbestimmung erfolgte auf viskosimetrischem Wege. Das erfindungsgemäß eingesetzte PHMB mit den genannten mittleren Molekulargewichten ist eine lediglich minimal toxische wasserlösliche Substanz, die weitestgehend von toxischen niedermolekularen Bestandteilen des PHMB bzw. dessen Synthesevorstufen oder dessen Derivaten befreit ist.

Die Herstellung des erfindungsgemäß verwendeten Poly(hexamethylen)biguanids erfolgt dadurch, daß man in an sich bekannter Weise Poly(hexamethylen)biguanid herstellt, z.B. wie es in der DE-PS 16 20 938 oder der GB-PS 12 02 495, auf deren Offenbarung hiermit in vollem Umfang Bezug genommen wird, beschrieben ist, und in an sich bekannter Weise den unerwünschten Molekulargewichtsanteil aus dem erhaltenen PHMB abtrennt, z.B. durch Dialyse, Molekularfiltration, HPLC, Gel-Permeations-Chromatographie (GPC), fraktionierte Fällung und dergleichen.

Die Herstellung kann auch so durchgeführt werden, daß man aus einem handelsüblichen PHMB, wie z.B. aus Vantocil^{R} IB oder Arlagard E oder anderen Handelsprodukten, die unerwünschte toxische, niedermolekulare Fraktion in der vorstehend beschriebenen Weise abtrennt.

Das erfindungsgemäß eingesetzte PHMB liegt in freier Form oder in Form eines wasserlöslichen Salzes, z.B. als Hydrochlorid, als Pulver (z.B. gefriergetrocknet) in 100 %iger Konzentration oder in wässriger Lösung vor. Es ist in Konzentrationen bis 40 Gew.-%, z.B. von 2 bis 40 Gew.-%, vorzugsweise 3 bis 30 Gew.-%, insbesondere 4 bis 20 Gew.-%, z.B. 4 Gew.-%, 4,5 Gew.-%, 5 Gew.-%, 6 Gew.-% oder 20 Gew.-% in der wässrigen Lösung (d.h. als Konzentrat) verfügbar.

Unter "PHMB" wird hierin sowohl das Poly(hexamethylen)biguanid als auch das Poly(hexamethylen)biguanid in Form eines wasserlöslichen Salzes verstanden.

Die Konzentration, in der das erfindungsgemäß verwendete PHMB bzw. die wässrige Lösung des PHMB in den erfindungsgemäßen Antiinfektiva, wie z.B. antimikrobiellen Mitteln enthalten ist, hängt von dem beabsichtigten Verwendungszweck des erfindungsgemäßen Antiinfektivum ab. Geeignete Konzentrationen liegen im allgemeinen im Bereich von 0,001 bis 0,05 Gew.-%, vorzugsweise im Bereich von 0,004 bis 0.03 Gew.-%, insbesondere im Bereich von 0,005 bis 0,012 Gew.- %, beispielsweise 0,005 Gew.-%, 0,006 Gew.-% oder 0,012 Gew.-%.

Die erfindungsgemäßen Antiinfektiva können die Oberflächenspannung herabsetzende Tenside, wie z.B. Polyethylenglycol, enthalten. Vorzugsweise wird Polyethylenglycol mit einem Molekulargewicht von 1.500 bis 6.000 und insbesondere ein solches Polyethylenglycol mit einem Molekulargewicht von 4.000, wie es unter dem Warenzeichen Lutrol^{R} E4.000 von der Firma BASF AG vertrieben wird, angewandt. Das Verhältnis von PHMB zu dem Tensid liegt geeigneterweise im Bereich von 6:1 bis 24:1, vorzugsweise im Bereich von 12:1 bis 22:1 und beträgt insbesondere 20:1.

Die erfindungsgemäßen Antiinfektiva können z.B. als Desinfektionsmittel, zur Prophylaxe und/oder Behandlung von Infektionen, als antimikrobielle Mittel, als antivirale Mittel, als antiparasitäre Mittel, als Wundantiseptika und/oder als Wundbehandlungsmittel angewandt werden und werden bevorzugt als Wundantiseptikum und/oder Wundbehandlungsmittel und/oder zur antimikrobiellen, antiviralen und/oder antiparasitären Behandlung eingesetzt. Sie können auf verschiedene Weise angewandt werden, wie z.B. lokal oder systemisch, oral, rektal, vaginal oder intravenös, vorzugsweise intravenös. Sie finden bei Mensch und Tier, vorzugsweise bei Menschen Anwendung. Bevorzugt ist ihre Verwendung zur antimikrobiellen, antiviralen und/oder antiparasitären Behandlung durch intravenöse Verabreichung, insbesondere zur antimikrobiellen Behandlung durch intravenöse Verabreichung.

Abhängig von der vorgesehenen Verwendung können die erfindungsgemäßen Mittel in verschiedenen Anwendungsformen bzw. pharmazeutischen Zubereitungsformen vorliegen, wie z.B. in Form von wässrigen Lösungen (gegebenenfalls mit einem Gehalt an lactatfreier Ringerlösung oder Kochsalzlösung, vorzugsweise lactatfreier Ringerlösung), Emulsionen, Suspensionen, Gelen, Salben oder Cremes, Tabletten, Kapseln, Dragées, Suppositorien und dergleichen. In diesen Zubereitungsformen sind zusätzlich die zur Herstellung der jeweiligen Zubereitungsform notwendigen üblichen Hilfs- und Zusatzstoffe enthalten.

Die erfindungsgemäßen Mittel besitzen zusätzlich zu der besseren Wirksamkeit gegenüber den bekannten Mitteln auf Basis von PHMB mit niedrigerem Molekulargewicht, eine bessere Verträglichkeit und Gewebekompatibilität, zeigen keine Resistenzbildung, bewirken keine zentralnervösen Störungen (Lähmungserscheinungen) oder andere systemische Nebenwirkungen und sind weniger toxisch als die in der DE-OS 35 37 627 eingesetzten Desinfektionsmittel auf Basis von PHMB mit einem Molekulargewicht von 1.700 bis 2.500 sowie das Handelsprodukt Lavasept^{R} (PHMB, M_{w} 2.610). Während bei dem bisher verwendeten Handelsprodukt Lavasept^{R} mit PHMB (Polyhexanidium) mit einem mittleren Molekulargewicht M_{w} von 2.610 eine 10 %ige Hämolyse bereits bei 0,21 % im Blut erfolgte, tritt bei Verwendung von PHMB mit mittlerem Molekulargewicht M_{w} 4.000 eine 10 %ige Hämolyse erst bei 0,5 % im Blut auf.

Zur Verwendung als Wundantiseptikum und/oder als Wundbehandlungsmittel wird das erfindungsgemäß angewandte Poly(hexamethylen)biguanid bzw. die vorstehend genannte konzentrierte wässrige Lösung des PHMB mit Wasser, lactatfreier Ringerlösung oder Kochsalzlösung, vorzugsweise mit lactatfreier Ringerlösung, verdünnt. Für diese Verwendung geeignete Konzentrationen des erfindungsgemäßen PHMB in Wasser, lactatfreier Ringerlösung oder Kochsalzlösung liegen im allgemeinen im Bereich von 0,001 bis 0,05 Gew.-%, vorzugsweise im Bereich von 0,004 bis 0,03 Gew.-% und insbesondere im Bereich von 0,005 bis 0,012 Gew.-%, z. B. betragen sie 0,005 Gew.-%, 0,006 Gew.-% oder 0,012 Gew.-%. In dieser Konzentration zeigen die erfindungsgemäßen Mittel eine ausgezeichnete mikrobistatische bzw. mikrobizide Wirksamkeit gegen alle klinisch relevanten Keime, wie Staphylokokken, Coli, Pseudomonas, Proteus, Aerobier und Anaerobier, Pilze, Viren, Amöben, Protozoen, wie z.B. Leichmanien, und andere Parasiten, die auch in Gegenwart von Eiweiß praktisch nicht gemindert wird.

Die Kochsalzlösung kann als 0,4 bis 1,2 Gew.-%ige Lösung eingesetzt werden. Vorzugsweise wird jedoch physiologische, d.h. 0,9 %ige Kochsalzlösung angewandt.

Aufgrund der vorstehend genannten überraschenden Eigenschaften der erfindungsgemäßen Mittel können diese Mittel überall dort eingesetzt werden, wo bereits die bekannten Poly(hexamethylen)biguanid enthaltenden Desinfektionsmittel eingesetzt werden, wobei es jedoch zur Erzielung von vergleichbaren gewünschten Ergebnissen möglich ist, aufgrund der Eigenschaften des erfindungsgemäß angewandten PHMB die erfindungsgemäßen Antiinfektiva bzw. antimikrobiellen Mittel in erheblich geringeren Konzentrationen anzuwenden als die bekannten Desinfektionsmittel. Zudem ergibt sich für die erfindungsgemäßen Wundantiseptika und Wundbehandlungsmittel eine bessere Gewebeverträglichkeit und erheblich geringere Toxizität. Das erfindungsgemäß angewandte PHMB wird zudem in geringerem Maße resorbiert als die bisher für diese Zwecke verwendeten Poly(hexamethylen)biguanide. Auch bei längerer Anwendung der erfindungsgemäßen Lösung ist keine Veränderung der guten Gewebeverträglichkeit zu beobachten. So treten bei längerer Behandlung weder auf den Wunden noch auf der Haut lokale Reizerscheinungen auf. Die erfindungsgemäßen Mittel können daher als lokale Antiseptika und zur Wundbehandlung bzw. zur Wundheilung in vielen Gebieten der Medizin eingesetzt werden. So können die erfindungsgemäßen Lösungen bei den verschiedenen chirurgischen Infektionen, wie z.B. bei chronischen Knocheninfektionen, postoperativen Infektionsquellen nach Osteosynthese, bei Weichteilinfektionen, wie z.B. bei Operationswunden nach Appendektomie oder Abdominaleingriffen, Haut- und Subcutis- und tieferen Weichteilabszessen oder Hand- und Fingerinfektionen, bei Peritonitis und infektionsgefährdeten Abdominaleingriffen sowie bei verschiedenen Infektionen aus der zahnärztlichen Chirurgie, wie z.B. nach Entfernung infizierter Zähne und Zysten, Denditio difficilis, infizierten Zahnfleischtaschen oder Alveolen, Osteomyelitis des Kieferknochens und dentogenen Kieferhöhlenentzündungen angewandt werden, wie auch eine prophylaktische Anwendung als Wundspülung während der Operation das Einsatzgebiet sein kann.

Die Anwendung der erfindungsgemäßen Mittel kann in Form von Spülungen von Herden als Vorbereitung zur Herdausräumung, Wundspülungen, als peroperative Spülungen oder auch als Spülungen während der Operation, in Form von mit der erfindungsgemäßen lösung getränkten Tamponaten (z.B. in der zahnärztlichen Chirurgie) oder Löngetten (z.B. zur Abdeckung von postoperativ offengelassenen Wunden) und dergleichen erfolgen.

Beim therapeutischen Gebrauch wird eine intermittierende Anwendung bevorzugt, wobei infizierte Wunden ein- bis dreimal täglich entsprechend den therapeutischen Anforderungen mit dem erfindungsgemäßen Mittel getränkt oder gespült werden. Zudem kann das erfindungsgemäße Mittel auch mittels Spül-Saug-Drainage zur Spülung intern angewandt werden. Für die intraoperative Prophylaxe kann das erfindungsgemäße Mittel während der gesamten Operationsdauer zur Spülung des Operationsgebietes sowie zum Abreiben von Implantaten mit Kompressen eingesetzt werden, wobei ohne weiteres Mengen von bis zu 1 bis 2 l appliziert werden können.

Die erfindungsgemäße Lösung ist auch zur Anwendung bei Kontaktlinsen, z.B. Aufbewahrungslösung der Kontaktlinsen oder Spüllösung für Kontaktlinsen, und als Augenspüllösung geeignet.

Zur Prophylaxe und Behandlung von Infektionen bzw. zur antimikrobiellen Behandlung von Mensch und Tier durch intravenöse Verabreichung wird das erfindungsgemäß angewandte PHMB bzw. die vorstehend genannte konzentrierte wässrige Lösung des erfindungsgemäß angewandten PHMB mit Wasser, Kochsalzlösung oder lactatfreier Ringerlösung, vorzugsweise lactatfreier Ringerlösung verdünnt. Geeignete Konzentrationen des erfindungsgemäßen PHMB in Wasser, Kochsalz oder lactatfreier Ringerlösung liegen für diese Verwendung im allgemeinen im Bereich von 0,000001 bis 0,05 Gew.-%, vorzugsweise im Bereich von 0,0001 bis 0,03 Gew.-% und insbesondere im Bereich von 0,001 bis 0,01 Gew.-%, und betragen beispielsweise 0,00005 Gew.-%, 0,0005 Gew.-%, 0,005 Gew.-%, 0,0012 Gew.-% oder 0,01 Gew.-%. In dieser Konzentration zeigen die erfindungsgemäßen intravenös verabreichten Lösungen eine ausgezeichnete mikrobistatische bzw. mikrobizide Wirksamkeit gegen alle klinisch relevanten Keime, wie Staphylokokken, Coli, Pseudomonas, Proteus, Aerobier und Anaerobier, Pilze, Viren, Amöben, Protozoen, wie z.B. Leichmanien, und andere Parasiten, die auch in Gegenwart von Eiweiß praktisch nicht gemindert wird.

Für die intravenöse Verabreichung können die Lösungen auch die vorstehend genannten, die Oberflächenspannung herabsetzenden Tenside enthalten, sind jedoch vorzugsweise frei von diesen Tensiden. In diesen Lösungen können ferner gegebenenfalls zusätzlich übliche Elektrolyte sowie übliche Hilfs- und Zusatzstoffe enthalten sein. Diese intravenös zu verabreichenden Lösungen sind zur Prophylaxe und/oder zur Behandlung von Infektionen, die durch klinisch relevante Keime, wie Staphylokokken, Coli, Pseudomonas, Proteus, Anaerobier, Pilze und Viren hervorgerufen werden, geeignet. Besonders geeignet sind die zur Prophylaxe und Behandlung von mikrobiellen Infektionen, die durch Anaerobier, Aerobier, Pilze, Viren, Protozoen und andere Parasiten hervorgerufen werden. Bei Untersuchungen wurde überraschend gefunden, daß bei der intravenösen Verwendung der erfindungsgemäßen Lösungen Lähmungserscheinungen, d.h. zentralnervöse Störungen, wie sie bei Verwendung von Lavasept-Lösungen bzw. Lösungen, die PHMB mit einem mittleren Molekulargewicht M_{w} von 2.610, die auch niedermolekulare Bestandteile des PHMB beinhalten, enthalten, auftreten, nicht beobachtet werden. Diese erfindungsgemäßen Lösungen sind daher nicht nur lediglich minimal toxisch und weisen eine bessere Wirksamkeit auf. sondern sind auch überraschend erheblich besser verträglich als die beschriebenen bekannten Lösungen auf Basis PHMB mit mittlerem Molekulargewicht M_{w} von 1.700 bis 2.600.

Die intravenös zu verabreichenden Lösungen können geeigneterweise in einer Dosierung von 0,001 bis 20 ml/kg Körpergewicht, beispielsweise in Dosen von 0,01 ml/kg Körpergewicht oder 10 ml/kg Körpergewicht angewandt werden.

### Erläuterung der Figuren

- Fig. 1: veranschaulicht die Molekulargewichtsverteilung von erfindungsgemäß angewandtem PHMB mit M_{w} 4.000 und handelsüblichem PHMB mit M_{w} 2.600,
- Fig. 2: veranschaulicht Ergebnisse des Hämolysetests.

### Wege zur Ausführung der Erfindung

Die nachfolgenden Beispiele dienen der Erläuterung der vorliegenden Erfindung.

### Beispiel 1

Aus Poly(hexamethylen)biguanid mit einem mittleren Molekulargewicht M_{w} von 3.500, Polyethylenglycol 4.000 (Lutro^{R} E 4000, BASF AG) und Wasser wurde durch Vermischen derselben eine erfindungsgemäße Lösung der folgenden Zusammensetzung hergestellt:

| | |
|---|---|
| Poly(hexamethylen)biguanid-Hydrochlorid, M_{w} 3.500 | 6 Gew.-% |
| Polyethylenglycol, M_{w} 4.000 | 0,3 Gew.-% |
| Wasser | 93,7 Gew.-% |

Das angewandte Poly(hexamethylen)biguanid-Hydrochlorid wurde in an sich bekannter Weise durch fraktionierte Filtration aus dem handelsüblichen Poly(hexamethylen)biguanid-Produkt Vantocil^{R} IB oder Arlagard E der Firma ICI abgetrennt. Vantocil IB bzw. Arlagard E ist eine wässrige Lösung, die als Wirkstoff 20 % Poly(hexamethylen)biguanid-Hydrochlorid enthält.

### Beispiel 2

Zur Herstellung einer erfindungsgemäßen Lösung wurde Beispiel 1 wiederholt mit der Ausnahme, daß anstelle des PHMB-Hydrochlorids das entsprechende PHMB eingesetzt wurde.

### Beispiel 3

Zur Herstellung eines Wundantiseptikums und Wundbehandlungsmittels wurden 0,2 % der in Beispiel 1 oder Beispiel 2 hergestellten Lösung mit lactatfreier Ringerlösung auf eine Endkonzentration an PHMB (M_{w} 3.500) von 0,012 Gew.-% verdünnt.

### Beispiel 4

Aus PHMB mit einem Molekulargewicht von 3.500, Polyethylenglycol 4.000 und Wasser, wurde in der gleichen Weise wie in Beispiel 1 beschrieben durch Vermischen der Bestandteile eine erfindungsgemäße Lösung der folgenden Zusammensetzung hergestellt:

| | |
|---|---|
| Poly(hexamethylen)biguanid-Hydrochlorid, M_{w} 3.500 | 20 Gew.-% |
| Polyethylenglycol, M_{w} 4.000 | 1,0 Gew.-% |
| Wasser | 79 Gew.-% |

Als PHMB und Polyethylenglycol wurden die bereits in Beispiel 1 angewandten Produkte eingesetzt.

### Beispiel 5

Aus PHMB-Hydrochlorid und Polyethylenglycol, wie sie in den Beispielen 1 und 4 angewandt wurden, sowie Wasser, wurde durch Vermischen derselben, wie es in Beispiel 1 beschrieben wurde, eine erfindungsgemäße Lösung der folgenden Zusammensetzung hergestellt:

| | |
|---|---|
| Poly(hexamethylen)biguanid-Hydrochlorid, M_{w} 3.500 | 5 Gew.-% |
| Polyethylenglycol, M_{w} 4.000 | 0,3 Gew.-% |
| Wasser | 94,7 Gew.-% |

### Beispiel 6

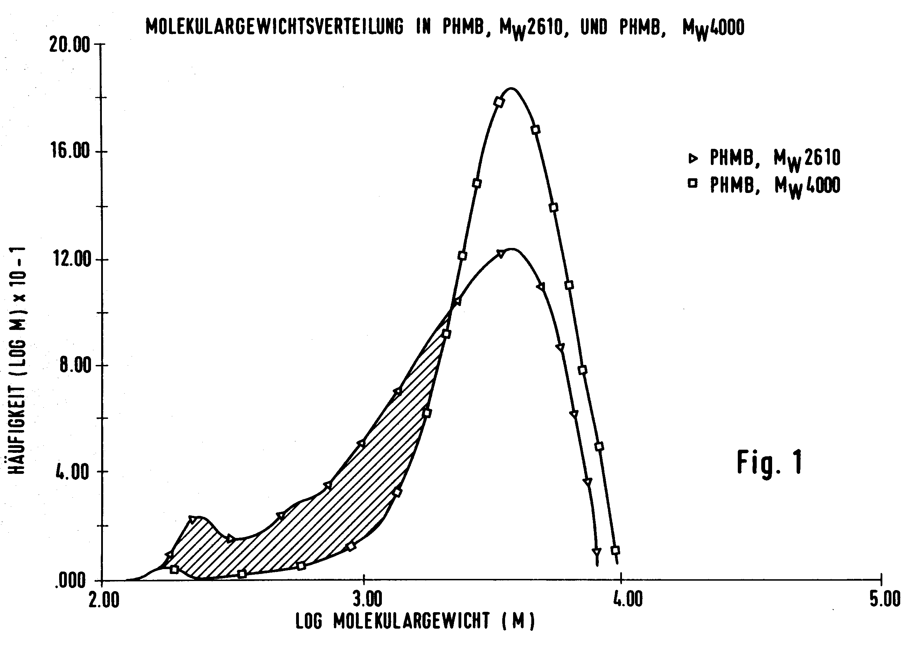

Zur Herstellung einer weiteren erfindungsgemäßen Lösung wurde Beispiel 1 wiederholt mit der Ausnahme, daß anstelle des dort verwendeten PHMB-Hydrochlorids, M_{w} 3500, PHMB mit einem mittleren Molekulargewicht M_{w} 4000, das ebenfalls wie in Beispiel 1 beschrieben hergestellt worden war, angewandt wurde. Aus Figur 1 ist die Molekulargewichtsverteilung von PHMB, M_{w} 4.000, im Vergleich zum handelsüblichen PHMB, M_{w} 2.600, ersichtlich.

### Beispiel 7

Zur Herstellung einer weiteren erfindungsgemäßen Lösung wurde Beispiel 4 wiederholt mit der Ausnahme, daß anstelle des dort verwendeten PHMB-Hydrochlorids mit M_{w} 3.500 ein in gleicher Weise hergestelltes PHMB-Hydrochlorid mit M_{w} 5.000 angewandt wurde.

### Beispiel 8

Gemäß diesem Beispiel wurde zu Vergleichszwecken die mikrobizide Wirkung des erfindungsgemäß eingesetzten PHMB mit einem Molekulargewicht von M_{w} 3.500 der mikrobiziden Wirkung von Vantocil^{R} IB gegenübergestellt. Alle Untersuchungen wurden gemäß der DGHM-Richtlinie I/2.3 (quantitative Suspension) durchgeführt. Die lg-Reduktionsfaktoren (Rf) sind aus Einzelwerten gemittelt. In diesen Tests wurde stets mit 3% Tween 80 + 3% Saponin + 0,1% Histidin + 0,1% Cystein inaktiviert. Die bei diesen Untersuchungen erhaltenen Ergebnisse sind in der nachfolgenden Tabelle 1 zusammengestellt. Dabei bedeuten:

| | |
|---|---|
| A | Vantocil^{R} IB, 20 Gew.-%iges PHMB, M_{w} 2.610 |
| B | 5 Gew.-%ige Lösung von PHMB, M_{w} 2.610 |
| C | 6 Gew.-%ige wässrige Lösung von PHMB, M_{w} 3.500 |

Diese Lösungen wurden in der nachfolgend angegebenen Konzentration gegenüber den jeweiligen Testkeimen und unter Anwendung der ebenfalls angegebenen Einwirkungszeiten geprüft.

**Tabelle 1**

| a) Testkeim: S. aureus, Einwirkzeit 2 Minuten | | | |
|---|---|---|---|
| Konzentration (%) | Rf "A" | Rf "B" | Rf "C" |
| 0,2 | 2,0 | 1,0 | 2,0 |
| 0,1 | 1,1 | 0,95 | 1,1 |
| 0,05 | 1 | 0,85 | 1 |
| 0,01 | 0,95 | 0,8 | 0,9 |

| b) Testkeim: S. aureus, Einwirkzeit 30 Minuten | | | |
|---|---|---|---|
| Konzentration (%) | Rf "A" | Rf "B" | Rf "C" |
| 0,2 | >5 | >5 | >5 |
| 0,1 | >5 | >5 | >5 |
| 0,05 | 4,5 | 4,0 | 4,8 |
| 0,01 | 3,0 | 1,5 | 3,0 |

| c) Testkeim: P. aeruginosa, Einwirkzeit 1/2 Minute | | | |
|---|---|---|---|
| Konzentration (%) | Rf "A" | Rf "B" | Rf "C" |
| 0,2 | 0,3 | 0,0 | 0,3 |

| d) Testkeim: P. aeruginosa, Einwirkzeit 2 Minuten | | | |
|---|---|---|---|
| Konzentration (%) | Rf "A" | Rf "B" | Rf "C" |
| 0,2 | 1,5 | 0,2 | 1,5 |
| 0,1 | 1,4 | 0,0 | 1,4 |
| 0,01 | 0,2 | 0,0 | 0,2 |

| e) Testkeim: P. aeruginosa, Einwirkzeit 30 Minuten | | | |
|---|---|---|---|
| Konzentration (%) | Rf "A" | Rf "B" | Rf "C" |
| 0,2 | >5 | >5 | >5 |
| 0,1 | >5 | >5 | >5 |
| 0,05 | >5 | 2,8 | >5 |

### Beispiel 9

Ebenfalls zu Vergleichszwecken, jedoch unter Belastung wurden die folgenden quantitativen Suspensionstests, gemäß DGHM-Richtlinie I/2.3 durchgeführt. In den nachfolgenden Tabellen 2 und 3 sind die bei diesen Tests erhaltenen Ergebnisse zusammen mit den jeweils als Belastung eingesetzten Substraten und den Prüfkonzentrationen der jeweilig angewandten zu prüfenden bzw. zu vergleichenden Prüfsubstanzen zusammengestellt. Die lg-Reduktionsfaktoren sind aus Einzelwerten gemittelt und bei den Tests wurde stets mit 3% Tween 80 + 3% Saponin + 0,1% Histidin + 0,1% Cystein inaktiviert.

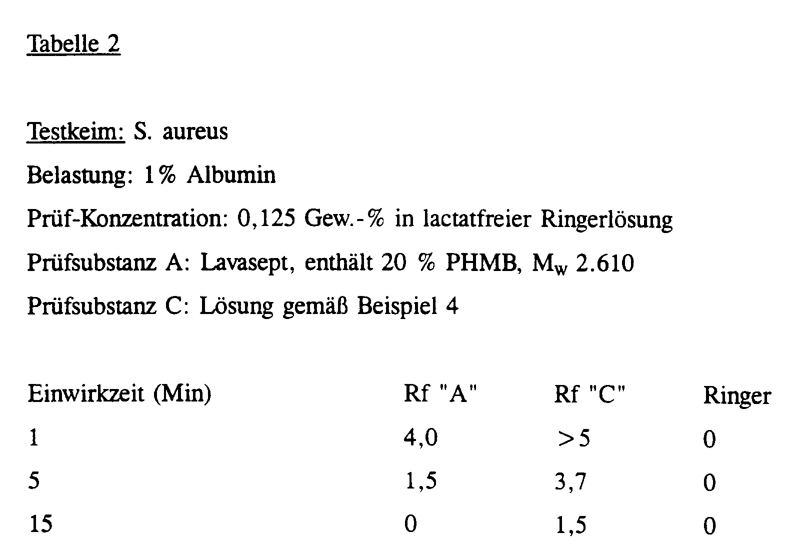

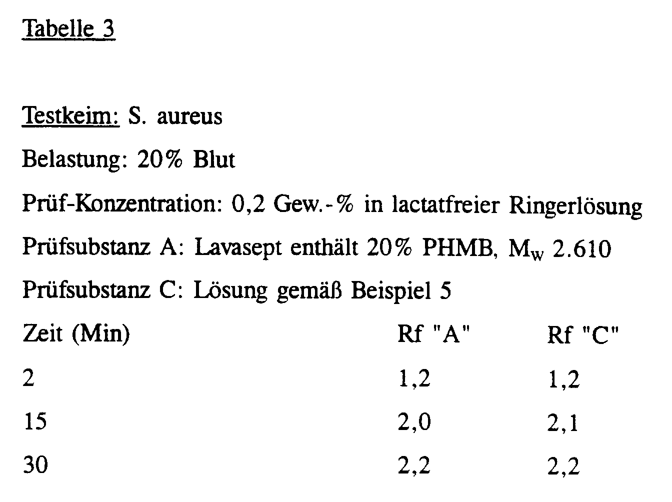

### Beispiel 10

Zum Nachweis der überraschenden Überlegenheit des erfindungsgemäß eingesetzten PHMB mit einem Molekulargewicht M_{w} von 3.500 gegenüber dem bekannten Lavasept mit einem PHMB mit einem Molekulargewicht M_{w} von 2.500 wurde das erfindungsgemäß angewandte PHMB, M_{w} 3.500, hinsichtlich der bakteriziden Wirksamkeit und der Toxizität dem PHMB mit einem M_{w} 2.500 gegenübergestellt. Die Untersuchungen zur Toxizität (Hämolysetest) wurden in folgender Weise durchgeführt:

Es wurde geprüft, ob bei Inkubation von Humanblut mit den Prüflösungen Hämoglobin freigesetzt wird. Folgende Lösungen wurden in Anlehnung an die Methode von H. Kreuzer, AMI-Berichte I/81, S. 14, geprüft:
- 1 ml frisches Citratblut und 1 ml 1 %ige Saponinlösung (in 0,9 %iger NaCl-Lösung gelöst)
- 1 ml frisches Citratblut und 1 ml 0,9 %ige NaCl-Lösung
- 1 ml frisches Citratblut und 1 ml Prüflösung
- 1 ml frisches Citratblut und 1 ml Ringer-Lösung.

Die Gemische wurden 45 Minuten bei 37 °C inkubiert und anschließend 5 Minuten bei 1000 g zentrifugiert. Der Hämoglobintest des Gemisches mit Saponin entspricht einer 100 %igen Hämolyse, der Hämoglobingehalt des Gemisches mit physiologischer NaCl-Lösung gilt als Null-Wert.

Bei diesen Untersuchungen wurde gefunden, daß die bakterizide Wirkung um das 3,3fache anstieg, wenn das Molekulargewicht des Poly(hexamethylen)biguanids von 2.500 auf 3.500 anstieg, während die zur Hämolyse notwendige Konzentration lediglich um das 2fache stieg. Die Hämolyse wurde durch die Untersuchung der hämolytischen Aktivität der beiden Poly(hexamethylen)biguanide mit unterschiedlichem Molekulargewicht unter Berücksichtigung ihrer bakteriziden Wirksamkeit bestimmt.

### Beispiel 11

Zum Nachweis der überraschenden Überlegenheit des erfindungsgemäß eingesetzten PHMB gegenüber dem bisher eingesetzten PHMB wurde ein PHMB mit einem mittleren Molekulargewicht M_{w} von 4.000 einem bisher angewandten PHMB mit einem Molekulargewicht von 2.610 (vgl. Figur 1 sowie Beispiel 6) gegenübergestellt. Die Untersuchungen zur Toxizität (Hämolysetest) wurden in der folgenden Weise durchgeführt:

Es wurde geprüft, ob bei Inkubation von Humanblut mit den Prütlösungen Hämoglobin freigesetzt wird. Folgende Lösungen wurden in Anlehnung an die Methode von H. Kreuzer, AMI-Berichte I/81, S. 14, geprüft:
- 1 ml frisches Citratblut und 1 ml 1 %ige Saponinlösung (in 0,9 %iger NaCl-Lösung gelöst)
- 1 ml frisches Citratblut und 1 ml 0,9 %ige NaCl-Lösung
- 1 ml frisches Citratblut und 1 ml Prüflösung
- 1 ml frisches Citratblut und 1 ml Ringer-Lösung.

Die Gemische wurden 45 Minuten bei 37°C inkubiert und anschließend 5 Minuten bei 1000 g zentrifugiert. Der Hämoglobintest des Gemisches mit Saponin entspricht einer 100 %igen Hämolyse, der Hämoglobingehalt des Gemisches mit physiologischer NaCl-Lösung gilt als Null-Wert.

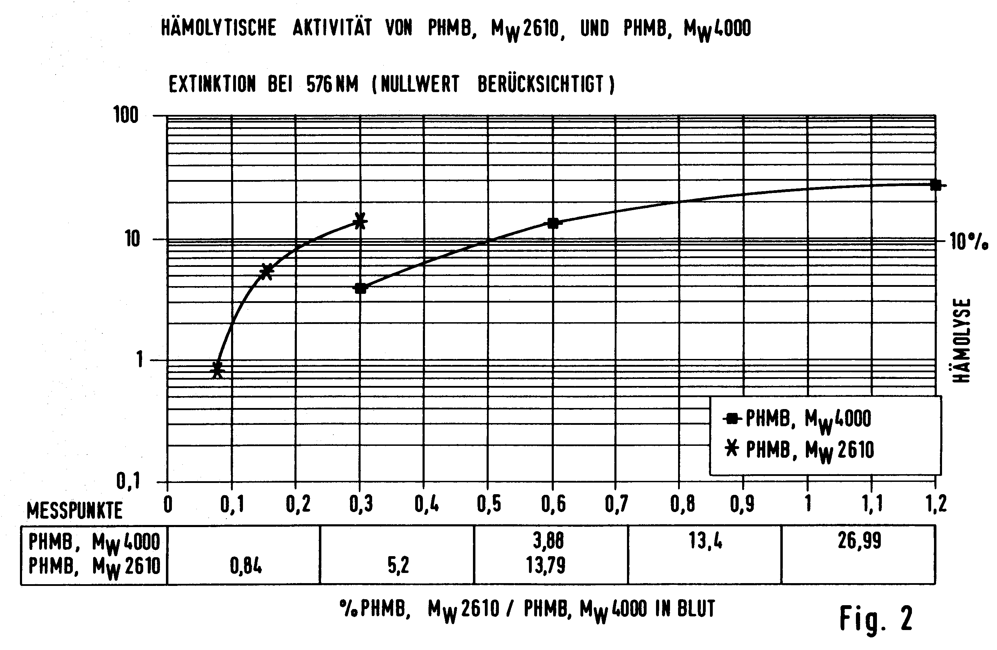

Die bei den Untersuchungen erhaltenen Ergebnisse sind aus der beiliegenden Figur 2 ersichtlich.

Die Ergebnisse zeigen, daß während bei dem bisher verwendeten PHMB, z.B Lavasept^{R} mit einem mittleren Molekulargewicht M_{w} 2.610 eine 10 %ige Hämolyse bereits bei 0,21 % im Blut erfolgte, bei Verwendung von PHMB (Lösung gemäß Beispiel 6) mit einem mittleren Molekulargewicht M_{w} 4.000 eine 10 %ige Hämolyse erst bei 0,5 % PHMB, M_{w} 4.000, im Blut erfolgte.

### Beispiel 12

Aus PHMB mit einem mittleren Molekulargewicht M_{w} 4.000 und Wasser wurde durch Vermischen derselben eine erfindungsgemäße Lösung der folgenden Zusammensetzung hergestellt:

| | |
|---|---|
| PHMB, M_{w} 4.000 | 4 Gew.-% |
| Wasser | 96 Gew.-%. |

Das angewandte PHMB wurde gemäß der in Beispiel 1 beschriebenen Weise erhalten.

### Beispiel 13

Aus PHMB mit einem mittleren Molekulargewicht M_{w} 5.000 und Wasser wurde durch Vermischen derselben eine erfindungsgemäße Lösung der folgenden Zusammensetzung hergestellt:

| | |
|---|---|
| PHMB, M_{w} 5.000 | 4,2 Gew.-% |
| Wasser | 95,8 Gew.-% |

Das angewandte PHMB wurde gemäß der im Beispiel 1 beschriebenen Weise erhalten.

### Beispiel 14

Aus PHMB mit einem mittleren Molekulargewicht M_{w} 4.500 und Wasser wurde durch Vermischen derselben eine erfindungsgemäße Lösung der folgenden Zusammensetzung hergestellt:

| | |
|---|---|
| PHMB, M_{w} 4.500 | 4,5 Gew.-% |
| Wasser | 95,5 Gew.-%. |

Das angewandte PHMB wurde gemäß der im Beispiel 1 beschriebenen Weise erhalten.

### Beispiel 15

Zur Herstellung einer weiteren erfindungsgemäßen Lösung wurde das Beispiel 1 wiederholt mit der Ausnahme, daß anstelle des dort angewandten PHMB mit einem mittleren Molekulargewicht M_{w} 3.500 PHMB mit einem mittleren Molekulargewicht M_{w} 4.000 verwendet wurde und der Gehalt an Polyethylenglycol (M_{w} 4.000) durch Wasser ersetzt wurde.

### Beispiel 16

Die Untersuchungen, wie sie in Beispiel 8 beschrieben wurden, wurden wiederholt, wobei jedoch als Testlösung "C" die in den Beispielen 12 bis 15 hergestellten Lösungen eingesetzt wurden. Bei diesen Untersuchungen wurden für die Lösungen der Beispiele 12 bis 15 Ergebnisse erhalten, die mit den in Beispiel 8 beschriebenen Ergebnissen vergleichbar waren.

### Beispiel 17

Aus PHMB mit einem mittleren Molekulargewicht M_{w} 4.000 und Wasser wurde durch Vermischen derselben eine erfindungsgemäße Lösung der folgenden Zusammensetzung hergestellt:

| | |
|---|---|
| PHMB, M_{w} 4.000 | 20 Gew.-% |
| Wasser | 80 Gew.-%. |

Das angewandte PHMB wurde gemäß der im Beispiel 1 beschriebenen Weise hergestellt.

### Beispiel 18

Die in Beispiel 9 beschriebenen Suspensionstests wurden wiederholt, wobei jedoch die folgenden Prüfsubstanzen eingesetzt wurden:
- Prüfsubstanz A:: Lavasept. enthaltend 20 % PHMB, M_{w} 2.610
- Prüfsubstanz C: : Lösung gemäß Beispiel 17
- Prüfsubstanz D:: Lavasept, wie es als Prüfsubstanz A verwendet wurde, jedoch ohne Gehalt an Polyethylenglycol.

Bei diesen Tests wurden Ergebnisse erhalten, die mit den in Beispiel 9 beschriebenen Ergebnissen vergleichbar waren.

### Beispiel 19

Zur Herstellung einer intravenös zu verabreichenden Lösung zur Prophylaxe und/oder Behandlung von Infektionen bzw. zur antimikrobiellen Behandlung wurden 0,2 % der in Beispiel 15 hergestellten Lösung mit lactatfreier Ringerlösung auf eine Endkonzentration an PHMB, M_{w} 4.000, von 0,0012 Gew.-% verdünnt.

### Beispiel 20

Zur Herstellung einer weiteren intravenös zu verabreichenden Lösung zur Prophylaxe und/oder Behandlung von Infektionen bzw. zur antimikrobiellen Behandlung wurden 0,2 % der in Beispiel 6 hergestellten Lösung mit lactatfreier Ringerlösung auf eine Endkonzentration an PHMB, M_{w} 4.000, von 0,01 Gew.-% verdünnt.

### Beispiel 21

Zur Herstellung einer weiteren erfindungsgemäßen intravenös zu verabreichenden Lösung wurden 0,2 % der in Beispiel 14 hergestellten Lösung mit 0,9 %iger Kochsalzlösung auf eine Endkonzentration an PHMB, M_{w} 4.500, von 0,005 Gew.-% verdünnt.

### Beispiel 22

Zur Herstellung einer weiteren erfindungsgemäßen intravenös zu verabreichenden Lösung wurden 0,2 % der in Beispiel 13 hergestellten Lösung mit Wasser auf eine Endkonzentration an PHMB, M_{w} 5.000, von 0,00005 Gew.-% verdünnt.

### Beispiel 23

Zur Herstellung einer weiteren intravenös zu verabreichenden Lösung wurden 0,2 % der in Beispiel 12 hergestellten Lösung mit lactatfreier Ringerlösung auf eine Endkonzentration an PHMB, M_{w} 4.000, von 0,0005 Gew.-% verdünnt.

### Beispiel 24

Zu Vergleichszwecken wurden die folgenden Lösungen, die hinsichtlich ihrer bioziden Wirksamkeit vergleichbar sind, als intravenöse Lösungen untersucht:
- Lösung A:: Lavasept 0,2 % in Ringerlösung (Konzentration PHMB, M_{w} 2.610: 0,04 %)
- Lösung B:: Lösung gemäß Beispiel 20 (Konzentration von PHMB, M_{w} 4.000: 0,01 %).

Die Untersuchungen wurden an Wistar-Ratten durchgeführt. Den Ratten wurden die zu untersuchenden Lösungen einmalig, langsam und kontinuierlich in eine Schwanzvene injiziert. Die Ratten wurden in drei verschiedene Gruppen zu je 10 Tieren (je 5 männliche Tiere und 5 weibliche Tiere) eingeteilt, wobei die
- Gruppe I: 5 ml/kg Körpergewicht der Lösung A,
- Gruppe II: 10 ml/kg Körpergewicht der Lösung A und
- Gruppe III: 10 ml/kg Körpergewicht der Lösung B
erhielt. Der Beobachtungszeitraum betrug 14 Tage, wobei Prüfungen 10 Minuten, 1 Stunde, 2 Stunden, 6 Stunden und 24 Stunden nach Verabreichung und dann bis zum Ende der 14tägigen Untersuchungsperiode täglich vorgenommen wurden.

Die Untersuchungen ergaben, daß während bei allen Gruppen vergleichbare Gewichtszunahmen erfolgten, bei Gruppe 1 bis 2 Stunden nach Verabreichung und bei Gruppe II bis 6 Stunden nach Verabreichung ein typisches Bild von Lähmungserscheinungen (zentralvenöse Störungen) auftrat: stark reduzierte Aktivität, Abdominal- oder Hockposition, abnormaler Gang, abnormale Körperhaltung, herabgesetzter Körper- und Abdominaltonus. Bei der Gruppe III wurden keine Veränderungen beobachtet. Das typische Bild von Lähmungserscheinungen, wie es bei den Gruppen I und II beobachtet wurde, trat bei Gruppe III nicht auf.

### Beispiel 25

### Herstellung eines Gels:

Aus den folgenden Bestandteilen wurde in üblicher Weise ein erfindungsgemäßes Gel hergestellt:

| | |
|---|---|
| Ringerlösung (lactatfrei) | 965,5 g |
| PHMB gemäß Beispiel 4 | 2,0 g |
| Hydroxyethylzellulose (DAB) | 32,5 g. |

### Beispiel 26

Zur Herstellung eines weiteren erfindungsgemäßen Gels wurde Beispiel 25 wiederholt mit der Ausnahme, daß anstelle der PHMB-Lösung gemäß Beispiel 4 die PHMB-Lösung gemäß Beispiel 17 eingesetzt wurde.

### Beispiel 27

Aus PHMB mit einem mittleren Molekulargewicht M_{w} 6.000 und Wasser wurde durch Vermischen derselben eine erfindungsgemäße Lösung der folgenden Zusammensetzung hergestellt:

| | |
|---|---|
| PHMB, M_{w} 5.000 | 4,2 Gew.-% |
| Wasser | 95,8 Gew.-%. |

Das angewandte PHMB wurde gemäß der im Beispiel 1 beschriebenen Weise erhalten.

### Beispiel 28

Zur Herstellung einer intravenös zu verabreichenden Lösung zur Prophylaxe und/oder Behandlung von Infektionen bzw. zur antimikrobiellen Behandlung wurden 0,2 % der in Beispiel 27 hergestellten Lösung mit lactatfreier Ringerlösung auf eine Endkonzentration an PHMB, M_{w} 6.000, von 0,0012 Gew.-% verdünnt.

## Patentansprüche

1. Antiinfektivum zur Behandlung von Mensch und Tier auf Basis von Poly(hexamethylen)buguanid, dadurch gekennzeichnet, daß das Poly(hexamethylen)biguanid ein mittleres Molekulargewicht M_{w} von 2.900 bis 15.000 aufweist.

2. Antiinfektivum nach Patentanspruch 1, dadurch gekennzeichnet, daß das mittlere Molekulargewicht M_{w} im Bereich von 3.000 bis 8.000 liegt.

3. Antiinfektivum nach Patentanspruch 2, dadurch gekennzeichnet, daß das Poly(hexamethylen)biguanid ein mittleres Molekulargewicht M_{w} im Bereich von 3.200 bis 5.000 aufweist.

4. Antiinfektivum nach einem der Patentansprüche 1 bis 3, dadurch gekennzeichnet, daß es in Form eines Wundantiseptikums und/oder eines Wundbehandlungsmittels vorliegt.

5. Antiinfektivum nach einem oder mehreren der Patentansprüche 1 bis 4, dadurch gekennzeichnet, daß es ein die Oberflächenspannung erniedrigendes Tensid enthält.

6. Antiinfektivum nach Patentanspruch 5, dadurch gekennzeichnet, daß es als Tensid Polyethylenglycol enthält.

7. Antiinfektivum nach Patentanspruch 6, dadurch gekennzeichnet, daß das Verhältnis von Poly(hexamethylen)biguanid zu Polyethylenglycol im Bereich von 6:1 bis 24:1 liegt.

8. Antiinfektivum nach Patentanspruch 7, dadurch gekennzeichnet, daß das Verhältnis im Bereich von 12:1 bis 22:1 liegt.

9. Antiinfektivum nach einem oder mehreren der Patentansprüche 1 bis 8, dadurch gekennzeichnet, daß es lactatfreie Ringerlösung oder Kochsalzlösung enthält.

10. Antiinfektivum nach einem oder mehreren der Patentansprüche 1 bis 9, dadurch gekennzeichnet, daß das Poly(hexamethylen)biguanid in einer Konzentration von 0,001-0,05 Gew.-% vorliegt.

11. Antiinfektivum nach Patentanspruch 10, dadurch gekennzeichnet, daß die Konzentration 0,004-0,03 Gew.-% beträgt.

12. Antiinfektivum nach Patentanspruch 11, dadurch gekennzeichnet, daß die Konzentration 0,005-0,012 Gew.-% beträgt.

13. Antiinfektivum nach einem oder mehreren der Patentansprüche 1 bis 12, dadurch gekennzeichnet, daß es in Form einer Kontaktlinsenaufbewahrungslösung und/oder Augenspüllösung vorliegt.

14. Antiinfektivum nach einem oder mehreren der Patentansprüche 1 bis 9, dadurch gekennzeichnet, daß es in Form einer intravenös zu verabreichenden Lösung vorliegt.

15. Antiinfektivum nach Patentanspruch 14, dadurch gekennzeichnet, daß das Poly(hexamethylen)biguanid in einer Konzentration von 0,000001 bis 0,05 Gew.-% vorliegt.

16. Antiinfektivum nach Patentanspruch 15, dadurch gekennzeichnet, daß die Konzentration 0,0001 bis 0,03 Gew.-% beträgt.

17. Antiinfektivum nach Patentanspruch 16, dadurch gekennzeichnet, daß die Konzentration 0.001 bis 0,01 Gew.-% beträgt.

18. Verwendung von Poly(hexamethylen)biguanid mit einem mittleren Molekulargewicht M_{w} von 2.900 bis 15.000 zur Herstellung eines Antiinfektivums zur Behandlung von Mensch und Tier.

19. Verwendung nach Patentanspruch 18, dadurch gekennzeichnet, daß das Poly(hexamethylen)biguanid ein mittleres Molekulargewicht M_{w} im Bereich von 3.000 bis 8.000 aufweist.

20. Verwendung nach Patentanspruch 19, dadurch gekennzeichnet, daß das mittlere Molekulargewicht M_{w} im Bereich von 3.200 bis 5.000 liegt.

## Claims

1. An anti-infective agent for human beings and animals, based on poly(hexamethylene)biguanide, characterised in that the poly(hexamethylene)biguanide features a mean molecular weight Mw of 2,900 to 15,000.

2. An anti-infective agent according to Patent Claim 1, characterised in that the mean molecular weight Mw lies in the range from 3,000 to 8,000.

3. An anti-infective agent according to Patent Claim 2, characterised in that the poly(hexamethylene)biguanide features a mean molecular weight Mw in the range from 3,200 to 5,000.

4. An anti-infective agent according to Patent Claims 1 to 3, characterised in that it is present in the form of a wound anti-septic agent and/or a wound treatment agent.

5. An anti-infective agent according to one of more of Patent Claims 1 to 4, characterised in that it contains a tenside for reducing surface tension.

6. An anti-infective agent according to Patent Claim 5, characterised in that it contains polyethylene glycol as the tenside.

7. An anti-infective agent according to Patent Claim 6, characterised in that the ratio of poly(hexamethylene)biguanide to polyethylene glycol lies in the range from 6:1 to 24:1.

8. An anti-infective agent according to Patent Claim 7, characterised in that the ratio lies in the range from 12:1 to 22:1.

9. An anti-infective agent according to one or more of Patent Claims 1 to 8, characterised in that it contains lactate-free Ringer solution or common salt solution.

10. An anti-infective agent according to one or more of Patent Claims 1 to 9, characterised in that the poly(hexamethylene)biguanide is present in a concentration of 0.001 - 0.05 % by weight.

11. An anti-infective agent according to Patent Claim 10, characterised in that the concentration is 0.004-0.03 % by weight.

12. An anti-infective agent according to Patent Claim 10, characterized in that the concentration is 0.005-0.12 % by weight.

13. An anti-infective agent according to Patent Claims 1 to 12, characterised in that it pertains in the form of a contact lens preservative solution and/or an eyewash solution.

14. An anti-infective agent according to Patent Claims 1 to 9, characterised in that it pertains in the form of a solution for intravenous administration.

15. An anti-infective agent according to Patent Claim 14, characterised in that the poly(hexamethylene)biguanide is present in a concentration from 0.000001 to 0.05 % by weight.

16. An anti-infective agent according to Patent Claim 15, characterised in that the concentration amounts to 0.0001 to 0.03 % by weight.

17. An anti-infective agent according to Patent Claim 16, characterised in that the concentration amounts to 0.001 to 0.01 % by weight.

18. The use of poly(hexamethylene)biguanide with a mean molecular weight Mw from 2,900 to 15,000, for the manufacture of an anti-infective agent for the treatment of human beings and animals.

19. Use in accordance with Patent Claim 18, characterised in that the poly(hexamethylene)biguanide features a mean molecular weight Mw in the range from 3,000 to 8,000.

20. Use in accordance with Patent Claim 19, characterised in that the mean molecular weight Mw lies in the range from 3,200 to 5,000.

## Revendications

1. Agent anti-infectieux pour le traitement d'un être humain et d'un animal, à base de poly(hexaméthylène)biguanide, caractérisé en ce que le poly(hexaméthylène)biguanide présente un poids moléculaire moyen M_{w} de 2.900 à 15.000.

2. Agent anti-infectieux selon la revendication 1, caractérisé en ce que le poids moléculaire moyen M_{w} se situe dans le domaine de 3.000 à 8.000.

3. Agent anti-infectieux selon la revendication 2, caractérisé en ce que le poly(hexaméthylène)biguanide présente un poids moléculaire moyen M_{w} dans le domaine de 3.200 à 5.000.

4. Agent anti-infectieux selon l'une quelconque des revendications 1 à 3, caractérisé en ce qu'il se présente sous la forme d'un antiseptique pour des blessures et/ou sous forme d'un agent de traitement des blessures.

5. Agent anti-infectieux selon une ou plusieurs des revendications 1 à 4, caractérisé en ce qu'il contient un agent tensioactif diminuant la tension superficielle.

6. Agent anti-infectieux selon la revendication 5, caractérisé en ce qu'il contient du polyéthylèneglycol à titre d'agent tensioactif.

7. Agent anti-infectieux selon la revendication 6, caractérisé en ce que le rapport du poly(hexaméthylène)biguanide au polyéthylèneglycol se situe dans le domaine de 6:1 à 24:1.

8. Agent anti-infectieux selon la revendication 7, caractérisé en ce que le rapport se situe dans le domaine de 12:1 à 22:1.

9. Agent anti-infectieux selon une ou plusieurs des revendications 1 à 8, caractérisé en ce qu'il contient une solution de chlorure de sodium ou une solution de Ringer exempte de lactate.

10. Agent anti-infectieux selon une ou plusieurs des revendications 1 à 9, caractérisé en ce que le poly(hexaméthylène)biguanide est présent en une concentration de 0,001-0,05% en poids.

11. Agent anti-infectieux selon la revendication 10, caractérisé en ce que la concentration s'élève de 0,004-0,03% en poids.

12. Agent anti-infectieux selon la revendication 11, caractérisé en ce que la concentration s'élève de 0,005-0,012% en poids.

13. Agent anti-infectieux selon une ou plusieurs des revendications 1 à 12, caractérisé en ce qu'il se présente sous la forme d'une solution de conservation de lentilles de contact et/ou d'une lotion oculaire.

14. Agent anti-infectieux selon une ou plusieurs des revendications 1 à 9, caractérisé en ce qu'il se présente sous la forme d'une solution à administrer par voie intraveineuse.

15. Agent anti-infectieux selon la revendication 14, caractérisé en ce que le poly(hexaméthylène)biguanide est présent en une concentration de 0,000001 à 0,05% en poids.

16. Agent anti-infectieux selon la revendication 15, caractérisé en ce que la concentration s'élève de 0,0001 à 0,03% en poids.

17. Agent anti-infectieux selon la revendication 16, caractérisé en ce que la concentration s'élève de 0,001 à 0,01% en poids.

18. Utilisation de poly(hexaméthylène)biguanide possédant un poids moléculaire moyen M_{w} de 2.900 à 15.000 pour la formulation d'un agent anti-infectieux destiné au traitement d'un être humain et d'un animal.

19. Utilisation selon la revendication 18, caractérisée en ce que le poly(hexaméthylène)biguanide présente un poids moléculaire moyen M_{w} dans le domaine de 3.000 à 8.000.

20. Utilisation selon la revendication 19, caractérisée en ce que le poids moléculaire moyen M_{w} se situe dans le domaine de 3.200 à 5.000.
